# EUROPEAN PATENT APPLICATION

(11) **EP 4 692 124 A1**
(43) Date of publication of application: **11.02.2026**
(21) Application number: 24785167.8
(22) Date of filing: 02.04.2024
(51) Int. Cl.: C07K 16/28, A61P 37/00, A61K 31/728, A61K 39/00

(54) **PHARMACEUTICAL COMPOSITION FOR PREVENTING OR TREATING SJOGREN'S SYNDROME, CONTAINING RECOMBINANT ANTI-SEMAPHORIN 4D ANTIBODY AS ACTIVE INGREDIENT**

(30) Priority: 03.04.2023 KR 20230043767; 12.01.2024 KR 20240005193
(71) Applicant: Sialbio Co., Ltd., Seoul 02455 (KR)
(72) Inventor: LEE, Sangwoo, Seoul 02734 (KR); PARK, Kyung Pyo, Seoul 04426 (KR)
(74) Representative: KATZAROV S.A.
(86) International application number: PCT/KR2024/004245
(87) International publication number: WO 2024/210462

(57) **Abstract**

The present invention pertains to a pharmaceutical composition for preventing or treating Sjogren's syndrome, the pharmaceutical composition containing a recombinant anti-Semaphorin 4D antibody as an active ingredient.

## Description

### [Technical Field]

The present disclosure relates to a pharmaceutical composition for preventing or treating Sjogren's syndrome, containing a recombinant anti-Semaphorin 4D antibody as an active ingredient.

### [Background Art]

Sjogren's syndrome is a rare autoimmune disease with a clearly unknown cause and characterized by decreased secretion of lachrymal glands and salivary glands, resulting in keratoconjunctivitis and xerostomia. For unknown reasons, autoimmune antibodies are produced and bind to exocrine tissues such as salivary glands and lachrymal glands to cause T cells and B cells to infiltrate into exocrine organs and induce a severe inflammatory response, ultimately destroying the tissues. Salivary dysfunction in Sjogren's syndrome gets worse over time, and the salivary gland failure is hardly restored, so that patients need to suffer from dry mouth continuously for life.

Meanwhile, there is yet disclosed no fundamental treatment for Sjogren's syndrome, and symptomatic treatment includes taking immunosuppressants such as prednisolone or prescribing pilocarpine, which artificially increases the amount of saliva by stimulating the parasympathetic nerves. However, when pilocarpine is applied, pilocarpine activates muscarinic receptors throughout the body to cause various side effects such as heart palpitations, dizziness, and hyperhidrosis, and is a temporary expedient that acinar cells that produce saliva are continuously destroyed, the destruction of the salivary glands is not prevented, and the remaining salivary gland acinar cells are stimulated to produce saliva as Sjogren's syndrome progresses. To this end, there are patients who do not experience a significant increase in a saliva amount even when taking pilocarpine, and in these cases, there are applied methods of replacing the function of saliva by using artificial saliva.

The reason why the development of salivary gland regenerative therapy for Sjogren's syndrome is difficult is that even if effective regenerative therapy is developed, a vicious cycle is repeated in which autoimmune antibodies in the blood bind to the regenerated salivary gland cells again to cause excessive infiltration of T and B cells and destroy the salivary glands again. Ultimately, a phenomenon of salivary dysfunction observed in Sjogren's syndrome is caused by various cytokines secreted by overactivated immune cells that have over-infiltrated into the salivary gland tissue, and thus there is currently no therapy capable of inhibiting the phenomenon. Therefore, there is an urgent need to develop a therapy that can suppress tissue infiltration and migration of immune cells and control the activation.

### [Disclosure]

### [Technical Problem]

In order to solve the problems, an object of the present disclosure is to provide a pharmaceutical composition for preventing or treating Sjogren's syndrome, containing a recombinant anti-Semaphorin 4D antibody as an active ingredient to restore the function of the salivary gland by locally injecting an anti-Semaphorin 4D antibody into the salivary gland to inhibit the infiltration of immune cells into the salivary gland caused by Sjogren's syndrome.

### [Technical Solution]

In order to achieve the objects, an aspect of the present disclosure provides a pharmaceutical composition for preventing or treating Sjogren's syndrome, pharmaceutical composition containing a recombinant anti-Semaphorin 4D antibody as an active ingredient.

In addition, the recombinant anti-Semaphorin 4D antibody may inhibit the expression level of at least one gene selected from CXCL12, CXCR4, or a combination thereof.

In addition, the recombinant anti-Semaphorin 4D antibody may block at least one protein selected from CD72, CD100, Plexin B2, or a combination thereof.

In addition, the composition may be applied in the form of an injection.

Another aspect of the present disclosure provides a kit for diagnosing Sjogren's syndrome containing an anti-Semaphorin 4D antibody as an active ingredient.

### [Advantageous Effects]

According to the exemplary embodiment of the present disclosure, the pharmaceutical composition for preventing or treating Sjogren's syndrome and the use thereof can treat hyposalivation by applying the recombinant anti-Semaphorin 4D antibody to effectively inhibit the migration of immune cells that causes destruction of salivary glands.

In addition, the pharmaceutical composition for preventing or treating Sjogren's syndrome can be applied to be delivered in the form of an injection to have no systemic side effects, such as heart palpitations, dizziness, and hyperhidrosis.

### [Description of Drawings]

FIG. 1A is a schematic diagram for describing a phenomenon in Sjogren's syndrome, FIG. 1B is a photograph showing results of staining a normal salivary gland and salivary gland biopsy tissues of a patient with Sjogren's syndrome, FIG. 1C is a graph showing RT-PCR results, and FIGS. 1D to 1G are diagrams and graphs showing Western blot results.
FIGS. 2A and 2B are graphs showing a correlation between a CXCL12 concentration and a sema 4D concentration in the saliva with Sjogren's syndrome.
FIG. 3A is a schematic diagram showing a system for co-culturing salivary gland epithelial cells or salivary gland organoids with Jurkat cells as a human CD4+ T cell line, and FIGS. 3B and 3C are diagrams showing results according to Experimental Example 3.
FIGS. 4A to 4C are diagrams showing results according to Experimental Example 4.
FIGS. 5A and 5B are diagrams showing results according to Experimental Example 5.
FIGS. 6A to 6E are diagrams showing results according to Experimental Example 6.
FIGS. 7A and 7B are diagrams showing results according to Experimental Example 7.
FIGS. 8A to 8C are diagrams showing results according to Experimental Example 8.
FIGS. 9A to 9E are diagrams showing results according to Experimental Example 9.
FIGS. 10A to 10F are diagrams showing results according to Experimental Example 10.

### [Best Mode]

Hereinafter, exemplary embodiments of the present disclosure will be described in detail. However, the present disclosure is not limited to exemplary embodiments disclosed below. The present disclosure may be implemented in various different forms, and the exemplary embodiments are just provided to complete the disclosure of the present disclosure and more accurately inform the contents of the present disclosure to those skilled in the art.

Hereinafter, a pharmaceutical composition for preventing or treating Sjogren's syndrome, containing a recombinant anti-Semaphorin 4D antibody as an active ingredient according to an exemplary embodiment of the present disclosure will be described in detail.

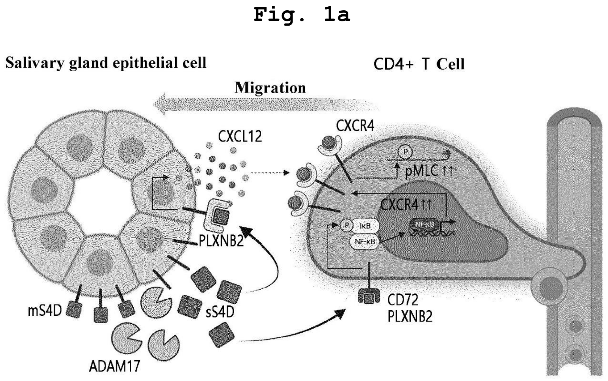

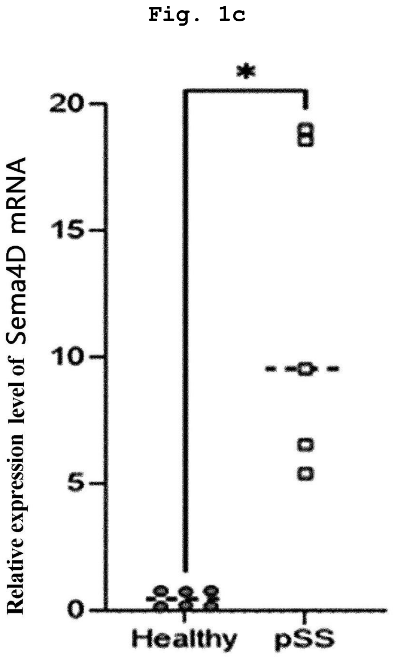

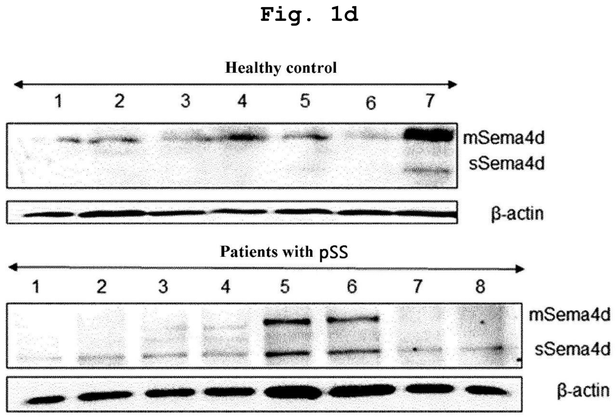

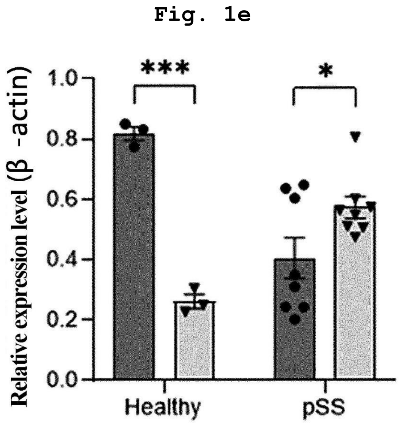

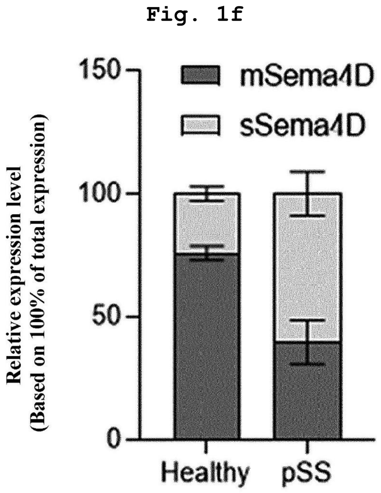

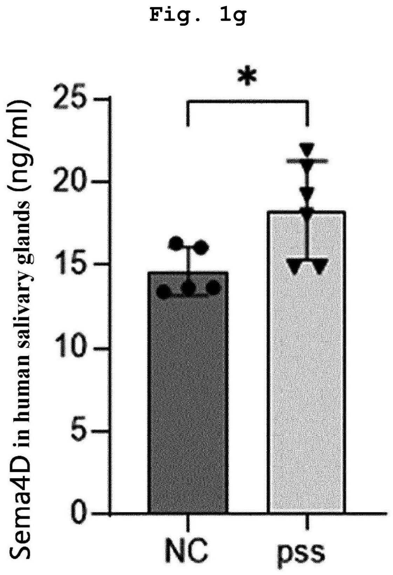

In the salivary gland epithelial tissue of Sjogren's syndrome, the expression of semaphorin 4D (sema 4D) is increased, and a large amount of cleaved sema 4D protein exists in the interstitial space. As shown in FIG. 1A, the large amount of sema 4D may bind to Plexin B2 of salivary gland epithelial cells to induce the salivary gland epithelial cells to overproduce CXCL12 which is an immune cell attractant. In addition, the large amount of sema 4D may bind to Plexin B2 and CD72 of CD4+ T cells to induce NF-kB activation, and as a result, may induce CD4+ T cells to overexpress CXCR4 which is a receptor for CXCL12. That is, a synergistic effect of CXCL12 overproduced in epithelial cells and CXCR4 overexpressed in CD4+ T cells may allow more immune cells to effectively infiltrate into epithelial cells.

In the exemplary embodiment, the above-described immune cells may effectively inhibit migration into salivary gland epithelial cells, thereby treating hyposalivation. Specifically, the immune cells may block one or more proteins selected from CD72, CD100, Plexin B2 or a combination thereof to inhibit the expression levels of one or more genes selected from CXCL12, CXCR4 or a combination thereof.

The pharmaceutical composition of the present disclosure may be applied in the form of an injection for local injection into the salivary gland, and in this case, in order to be formulated as the injection, the pharmaceutical composition may be prepared in the form of unit-dose ampoules or multiple dosages by mixing a preservative, an isotonic agent, an analgesic, a solubilizer, a buffer, a stabilizer, and the like using a pharmaceutically acceptable diluent. Accordingly, the pharmaceutical composition may have no systemic side effects such as heart palpitations, dizziness, hyperhidrosis, and the like.

In addition, the pharmaceutical composition of the present disclosure may include pharmaceutically acceptable additives, such as carriers, diluents, binders, disintegrants, lubricants, pH regulators, antioxidants, and solubilizers, within a range that does not interfere with the effects of the active ingredients. Examples of pharmaceutically acceptable additives that may be used to formulate the pharmaceutical composition of the present disclosure may be used with microcrystalline cellulose, xylitol, erythritol, methyl cellulose, polyvinylpyrrolidone, starch, acacia, alginate, gelatin, lactose, dextrose, sucrose, propylhydroxyben, zoate, cellulose, water, methylhydroxybenzoate, magnesium stearate, talc, sorbitol, mannitol, maltitol, calcium phosphate, calcium silicate, mineral oil, or the like.

The content of the active ingredient in the preparation according to the present disclosure may be appropriately selected depending on the absorption rate, inactivation rate, excretion rate of the active ingredient in the body, age, sex, and condition of a user, and the like. The active ingredient of the present disclosure may be administered in 0.0001 nM to 1000 mM, preferably 0.001 nM to 100 mM, and administered 1 to 3 times a day or several times for a predetermined period.

As used herein, the term "prevention" means all actions that inhibit or delay the occurrence, spread, and recurrence of Sjogren's syndrome by administration of the pharmaceutical composition according to the present disclosure.

A method for preventing or treating Sjogren's syndrome according to another exemplary embodiment of the present disclosure may include administering an effective amount of recombinant anti-Semaphorin 4D antibody to a subject in need thereof.

Another exemplary embodiment of the present disclosure provides a use of a recombinant anti-Semaphorin 4D antibody for use in the preparation of a pharmaceutical agent for the prevention or treatment of Sjogren's syndrome.

A kit for diagnosing Sjogren's syndrome according to another exemplary embodiment of the present disclosure may include an anti-Semaphorin 4D antibody as an active ingredient. Specifically, the kit for diagnosing Sjogren's syndrome may measure the amount of semaphorin 4D contained in the saliva or blood using ELISA, etc., and may determine whether Sjogren's syndrome is present based thereon.

A method for diagnosing Sjogren's syndrome according to another exemplary embodiment of the present disclosure may include measuring the expression or activity of semaphorin 4D by contacting an isolated subject sample with a recombinant anti-Semaphorin 4D antibody.

Another exemplary embodiment of the present disclosure provides a use of a recombinant anti-Semaphorin 4D antibody for use in the preparation of an agent for the diagnosis of Sjogren's syndrome.

Since the anti-Semaphorin 4D antibody has been described in detail above, it will be omitted.

Hereinafter, the present disclosure will be described in detail based on the following Experimental Examples. The following Experimental Examples are only intended to illustrate the present disclosure, and the scope of the present disclosure is not limited by the following Experimental Examples.

Hereinafter, in all experiments, CD100 (SEMA4D) Monoclonal Antibody (BMA12 (BMA-12)), eBioscience^{™} was used as an anti-Semaphorin 4D (sema 4D) antibody.

### Experimental Example 1: Sjogren's Syndrome Analysis

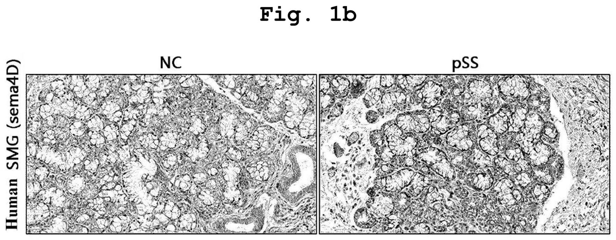

To identify the characteristics of patients with Sjogren's syndrome, a normal salivary gland and a minor salivary gland biopsy tissue of a patient with Sjogren's syndrome were observed under an electron microscope, and RT-PCR and Western blot were performed, and then the results were shown in FIGS. 1B to 1G.

Referring to FIGS. 1B to 1G, cleaved semaphorin 4D (sema 4D) was increased in the salivary gland epithelial cells of a patient with Sjogren's syndrome (hereinafter referred to as 'pss'), and the increased expression of sema 4D was observed in the salivary gland epithelial cells of the pss. The expression of sema 4D significantly increased in the salivary gland of the pss was also confirmed at the mRNA level. In addition, it was confirmed that most of the sema 4D in the normal salivary gland was present in a membrane-bound form, whereas the sema 4D in the salivary gland of the pss was present in a cleaved form, and it was found that the cleaved sema 4D was also secreted from the saliva of the patient.

### Experimental Example 2: Correlation between CXCL12 concentration and Sema 4D concentration

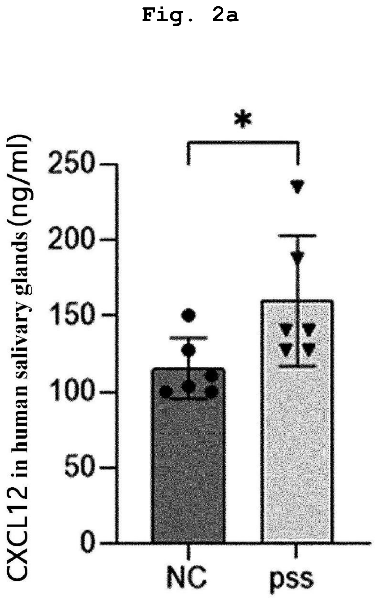

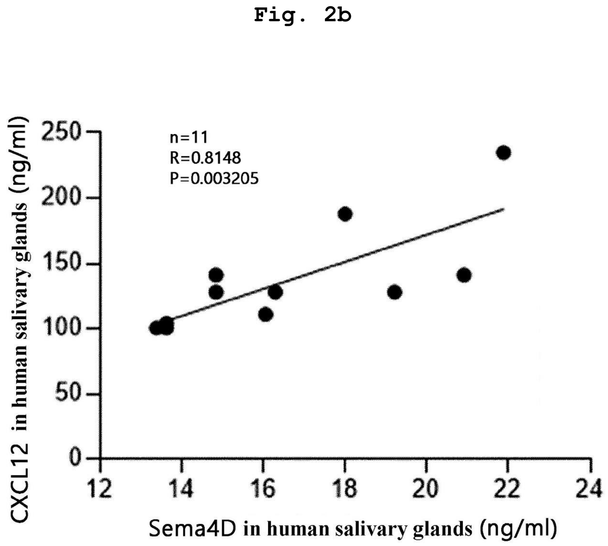

The concentrations of CXCL12 and semaphorin 4D (sema 4D) in the saliva of the pss were measured, and the results were shown in FIGS. 2A and 2B.

Referring to FIGS. 2A and 2B, it was confirmed that the concentration of sema 4D in the saliva with Sjogren's syndrome and the concentration of CXCL12 in the saliva as chemoattractant attracting immune cells were increased simultaneously, which indicated that the sema 4D and CXCL12 had a high correlation. Therefore, it was found that a high concentration of cleaved sema 4D in the salivary gland of the pss produced CXCL12 attracting the immune cells.

### Experimental Example 3: Relationship between Sema 4D and immune cell migration

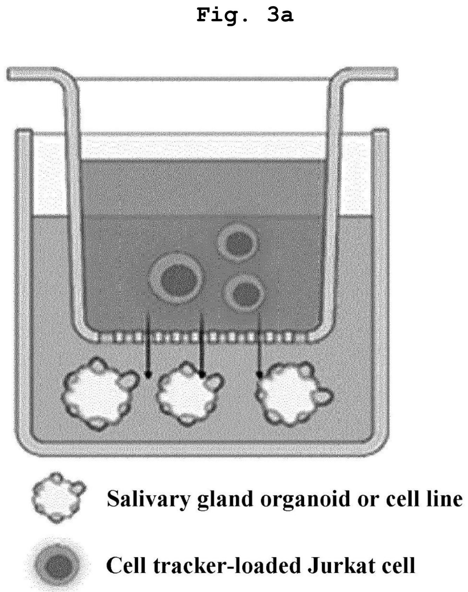

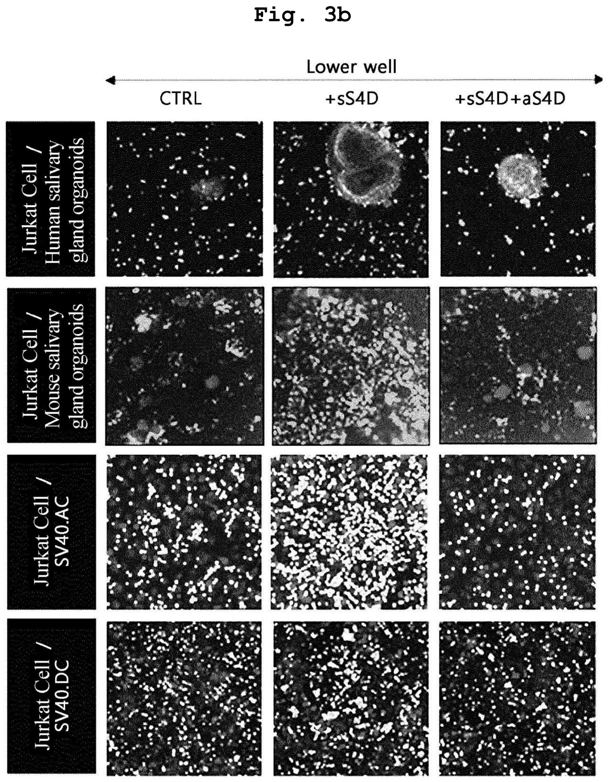

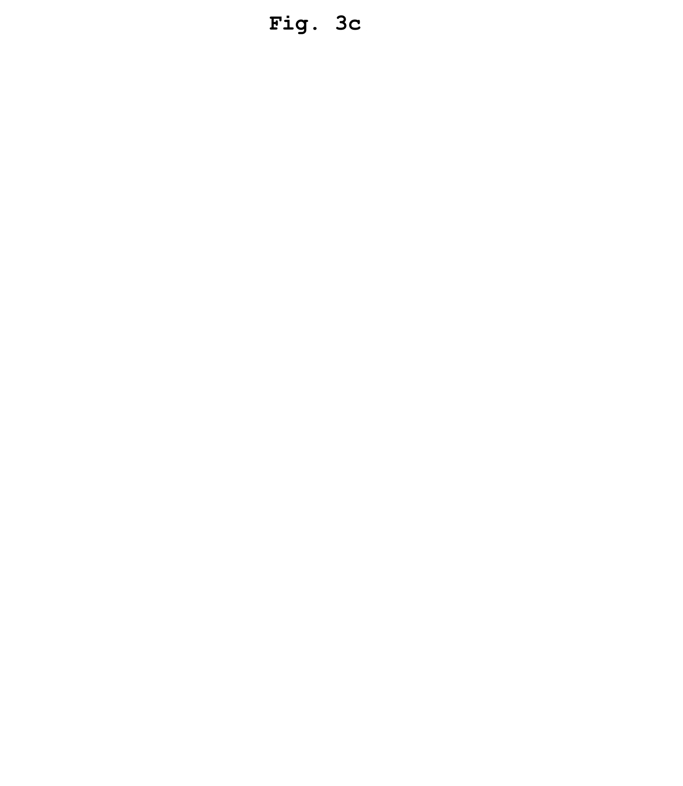

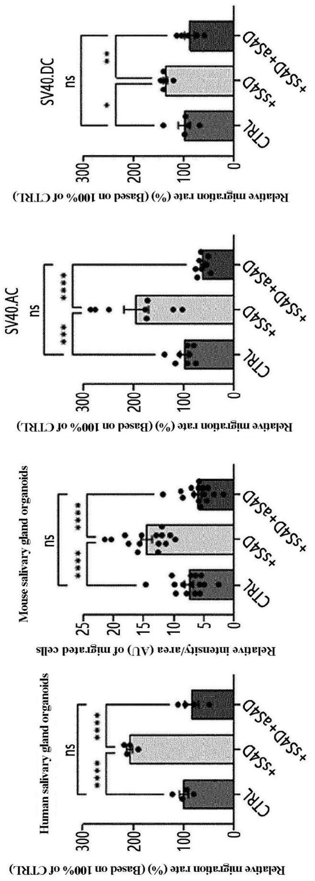

To reproduce a relationship between immune cells and salivary gland epithelial cells, a system was established as shown in FIG. 3A to co-culture salivary gland epithelial cells (NS-SV-DC, NS-SV-AC - human salivary gland ductal cell line and acinar cell line) or organoids (mouse salivary gland organoids) and Jurkat cells as a human CD4+ T cell line. Jurkat cells and salivary gland epithelial cells or tissues tagged with different fluorescence were planted in upper and lower chambers, respectively, and then the lower chamber was treated with recombinant sema 4D (+Ss4D) or treated with sema 4D and anti-sema 4D blocking antibody (+aS4D) together, and the results were shown in FIGS. 3B and 3C.

CD100 (SEMA4D) Monoclonal Antibody (BMA12 (BMA-12)), eBioscience^{™} was used as the anti-sema 4D blocking antibody.

Referring to FIGS. 3B and 3C, it was confirmed that in a group treated with recombinant sema 4D, approximately twice as many Jurkat cells migrated to the lower chamber as those in an untreated control group (CTRL), and in a group treated with anti-sema 4D together, the number of migrated immune cells decreased again to that of the control group (CTRL). Therefore, it was found that sema 4D promoted immune cell migration.

### Experimental Example 4: Relationship between Sema 4D and epithelial cells

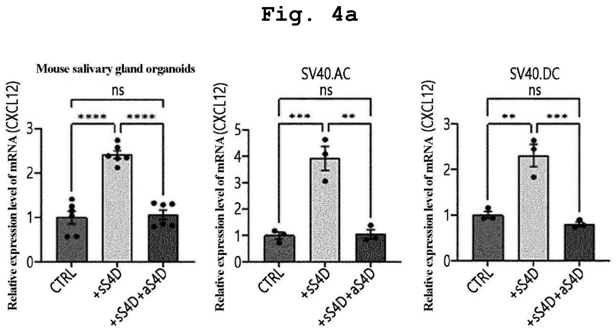

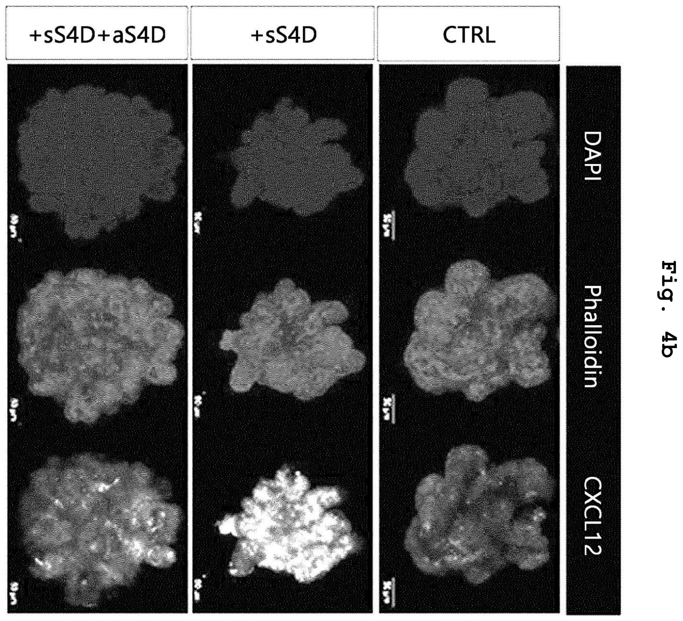

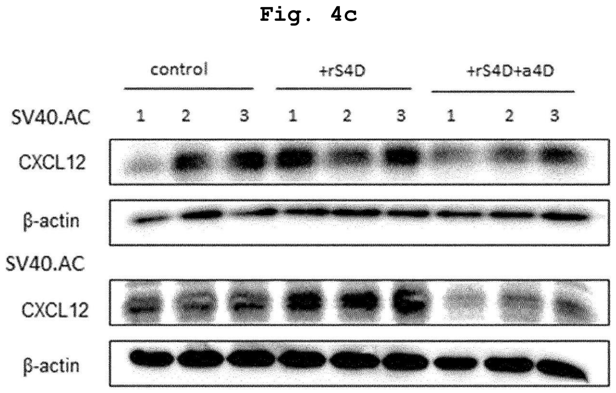

After salivary gland epithelial cell lines or organoids were treated with sema 4D, Western blot and immunofluorescence staining were performed, and the results were shown in FIGS. 4A to 4C.

Referring to FIGS. 4A to 4C, it was confirmed that the production of CXCL12 in the salivary gland epithelial cells increased.

### Experimental Example 5: Identification of receptors related with CXCL12

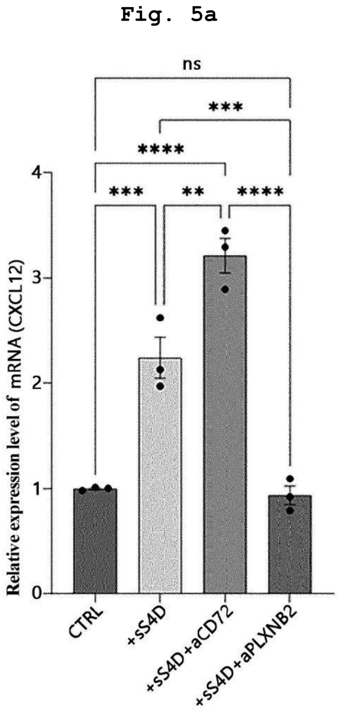

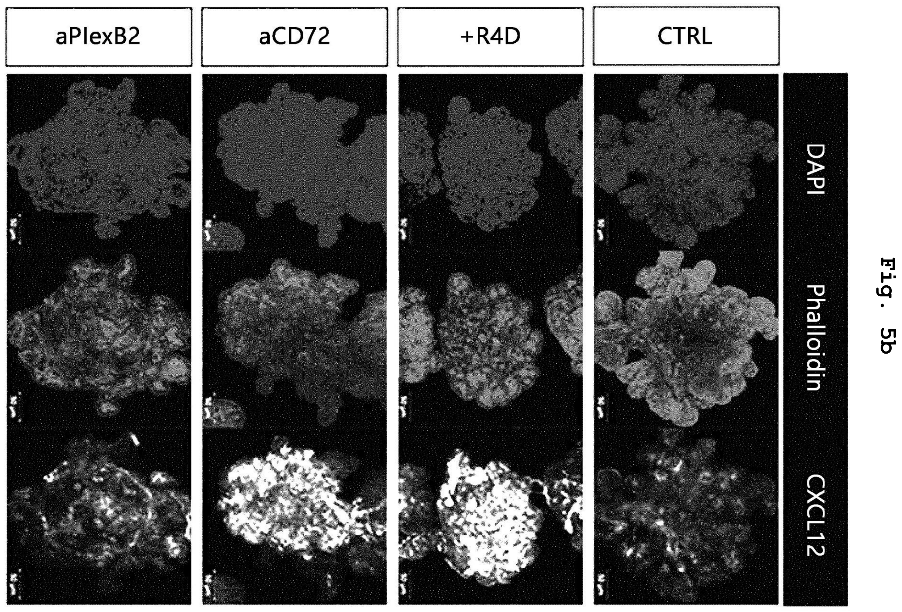

To determine which receptor mediated this response among CD72 and Plexin B2, as receptors for sema 4D, receptor blocking was attempted on each receptor using antibodies, and the results were shown in FIGS. 5A and 5B.

Referring to FIGS. 5A and 5B, it was confirmed that the CXCL12 expression increased by treatment with sema 4D was inhibited only in a group treated with sema 4D and an anti-Plexin B2 blocking antibody. Therefore, it could be seen that these responses were mediated by Plexin B2.

### Experimental Example 6: Confirmation of effect of Sema 4D on T cells

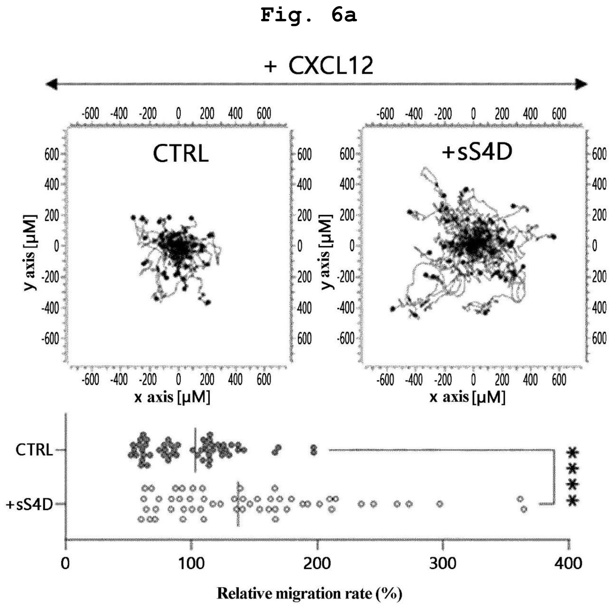

To determine whether semaphorin 4D (sema 4D) affected the migration of T cells, Jurkat cells were treated with sema 4D and then Western blot and immunofluorescence staining were performed, and the results were shown in FIGS. 6A to 6E.

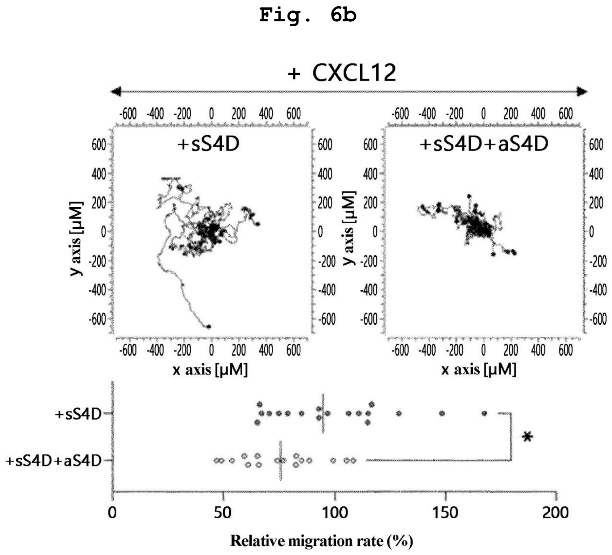

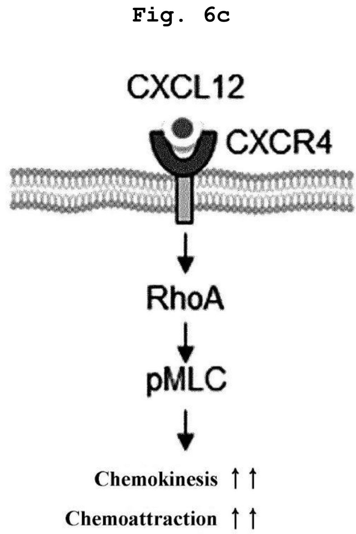

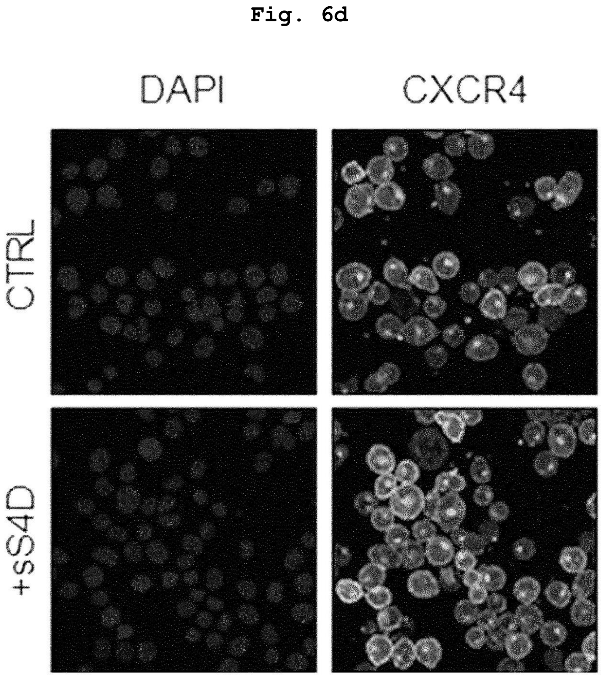

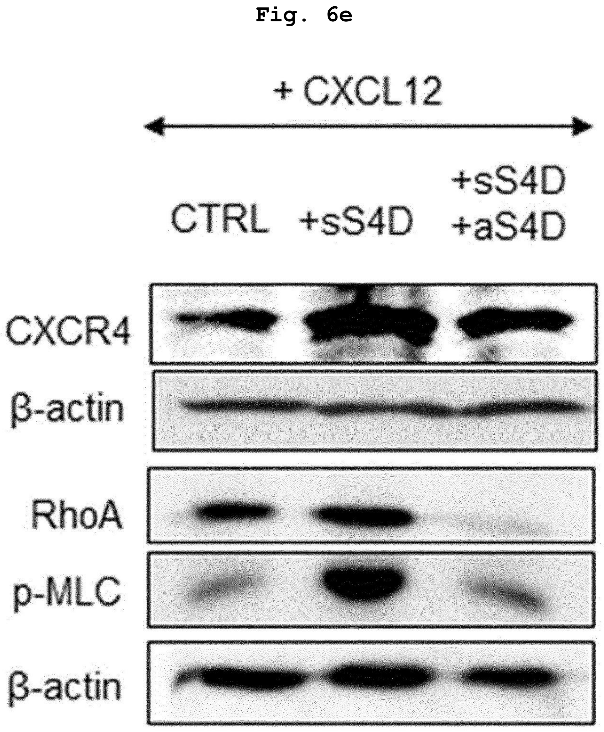

Referring to FIGS. 6A and 6B, it was confirmed that the migration rate of T cells increased, and the increased migration rate was decreased to a normal level by treatment with the anti-sema 4D blocking antibody. This was expected to be caused by cellular signaling from CXCR4 (receptor of CXCL12) -> RhoA -> phosphorylated myosin light chain, as shown in FIG. 6C. Referring to FIGS. 6D and 6E, in the Jurkat cells treated with sema 4D, the expression level of CXCR4 was increased, and RhoA and pMLC were also increased simultaneously. However, it was confirmed that these increased factors decreased again when treated with the anti-sema 4D blocking antibody.

### Experimental Example 7: Confirmation of correlation between CXCR4 and NF-kB (pNF-kb)

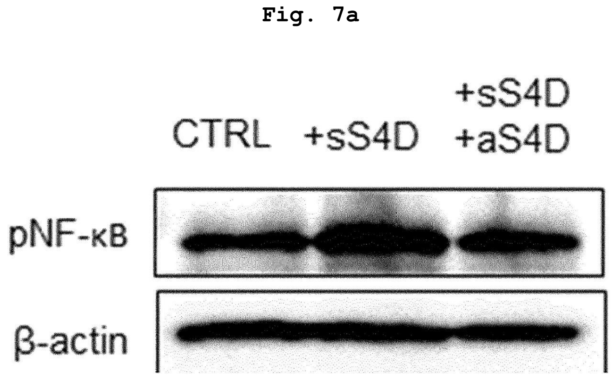

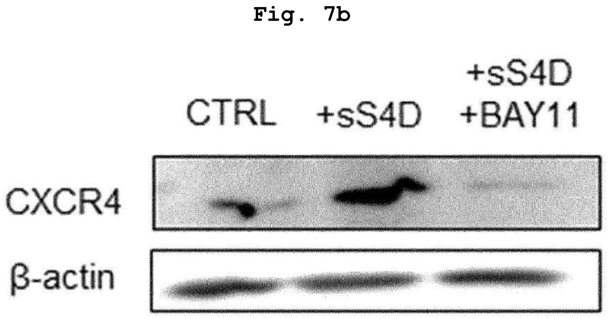

Based on the literature results showing that transcriptional regulation of CXCR4 was performed by NF-kB, to determine the increase in phosphorylated NF-kB (pNF-kb), Jurkat cells were treated with sema 4D and then Western blot was performed, and the results were shown in FIGS. 7A and 7B.

Referring to FIGS. 7A and 7B, it was confirmed that when the Jurkat cells were treated with sema 4D, the amount of pNF-Kb increased, and when BAY11, a specific inhibitor of NF-kb (a compound serving to inhibit an NF-NF-kb signaling pathway), was treated together, an increase in the expression level of CXCR4 caused by sema 4D was inhibited.

### Experimental Example 8: Identification of receptors related to CXCR4

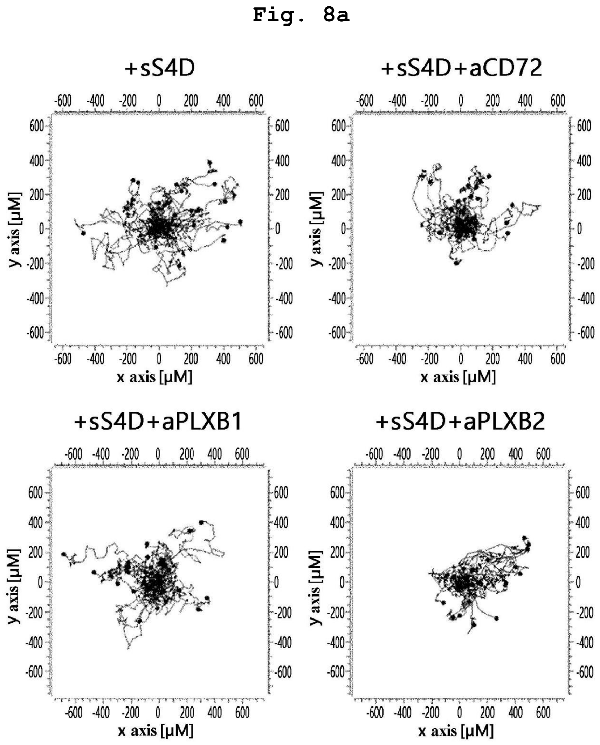

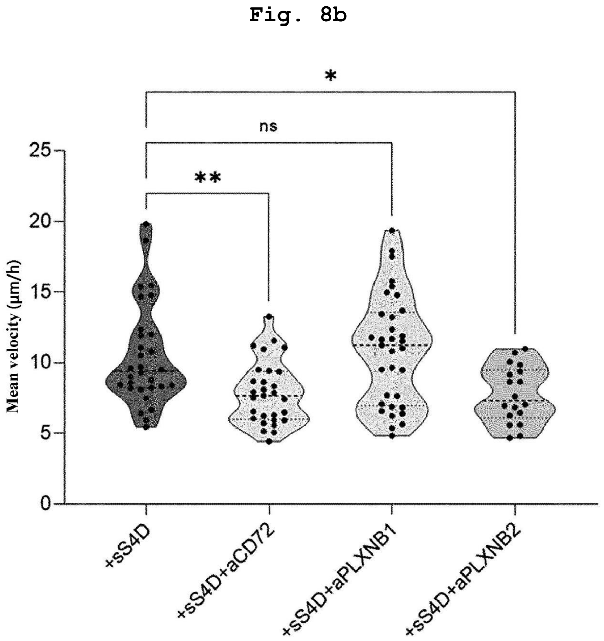

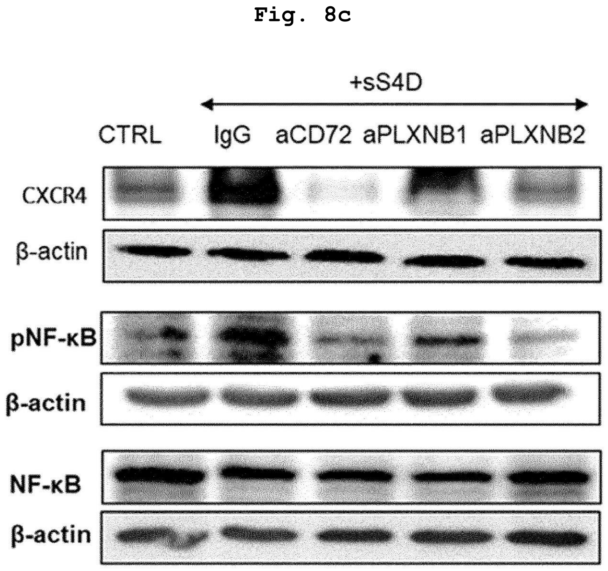

To determine which receptor mediated this response among CD72, Plexin B2, and Plexin B1, as receptors for sema 4D, receptor blocking was attempted on each receptor using antibodies, and the results were shown in FIGS. 8A to 8C.

Referring to FIGS. 8A to 8C, it was confirmed that CXCR4 was inhibited when treated with an anti-CD72 blocking antibody or anti-Plexin B2 blocking antibody. Therefore, it could be seen that these responses were mediated by CD72 and Plexin B2.

### Experimental Example 9: Confirmation of condition of mouse with Sjogren's syndrome

An experiment was performed to confirm the expression levels of immune cells, sema 4D, and CXCL12 using a Non-obese diabetic (NOD) mouse, which was a Sjogren's syndrome mouse model.

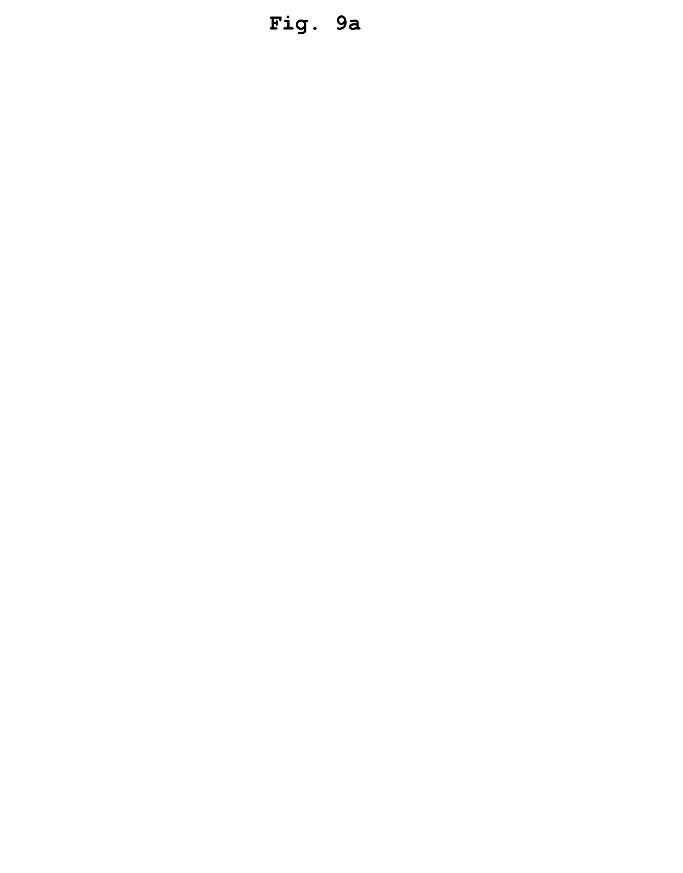

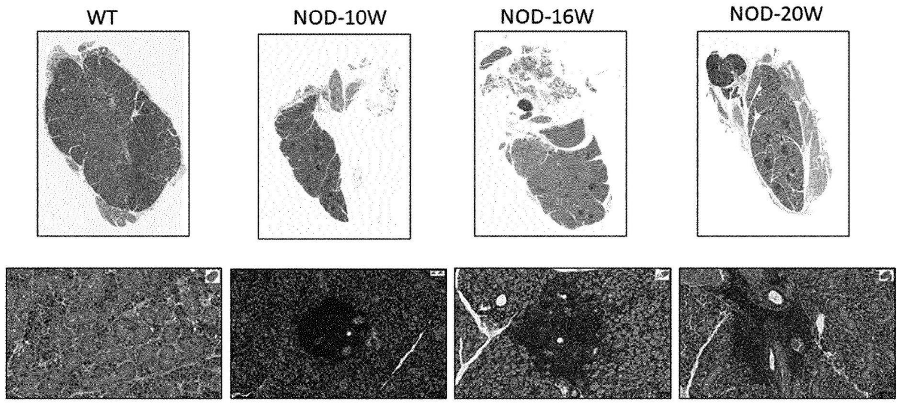

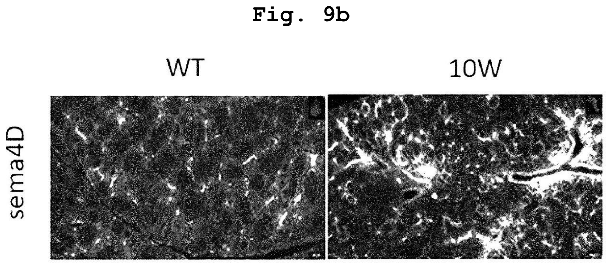

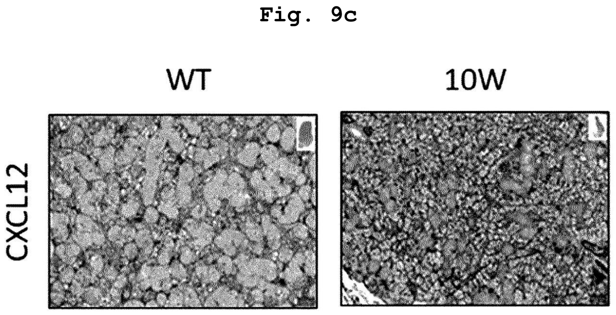

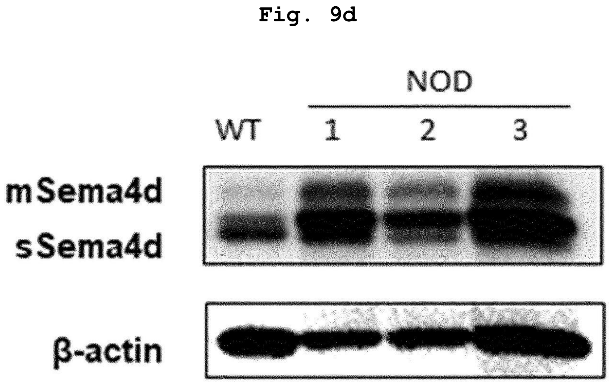

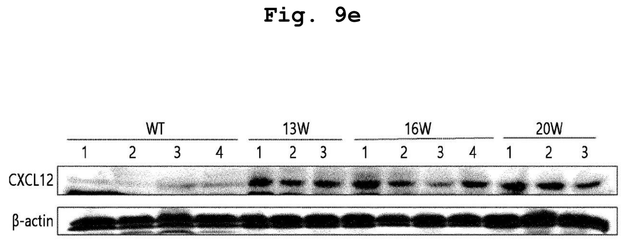

Referring to FIG. 9, symptoms of Sjogren's syndrome, such as immune cell infiltration into the salivary gland, salivary gland destruction, and decreased saliva volume, began to appear from 9 to 10 weeks, and immune cell infiltration foci began to appear from week 10. In addition, it was confirmed that the expression level of sema 4D and the amount of cleaved sema 4D within the tissues were increased, and the expression level of CXCL12 were also increased. Therefore, the same increase in sema 4D and CXCL12 observed in the pss was observed even in an NOD mouse.

### Experimental Example 10: Confirmation of treatment effect of Sjogren's syndrome

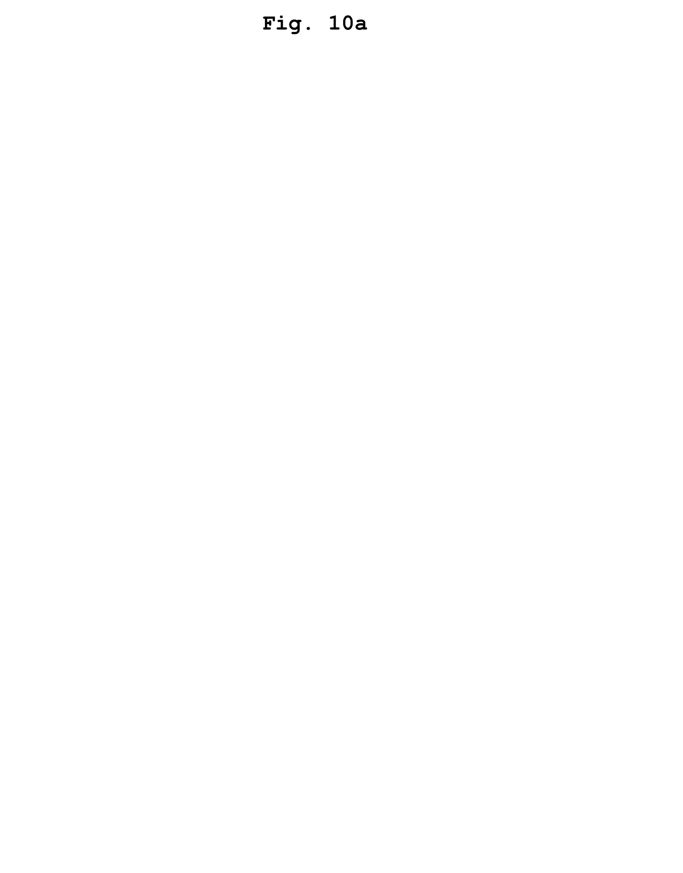

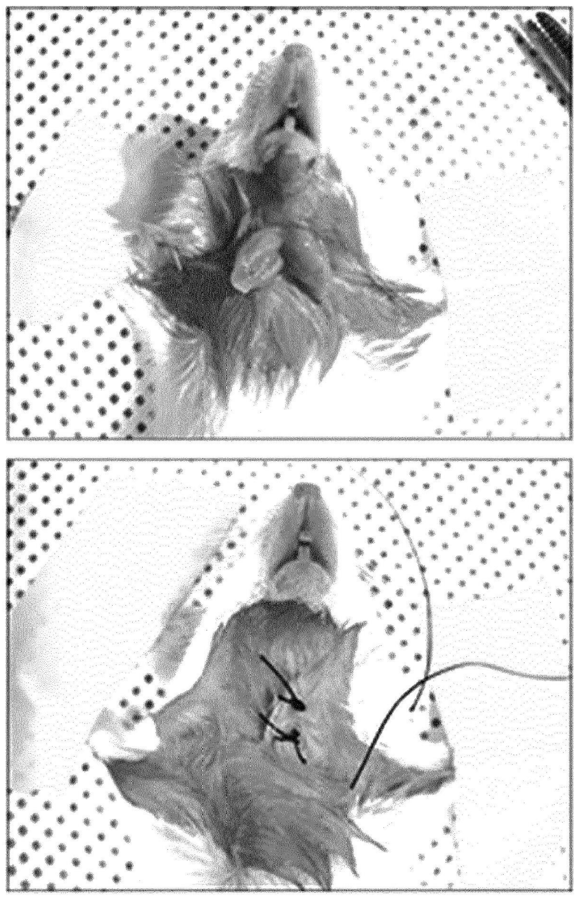

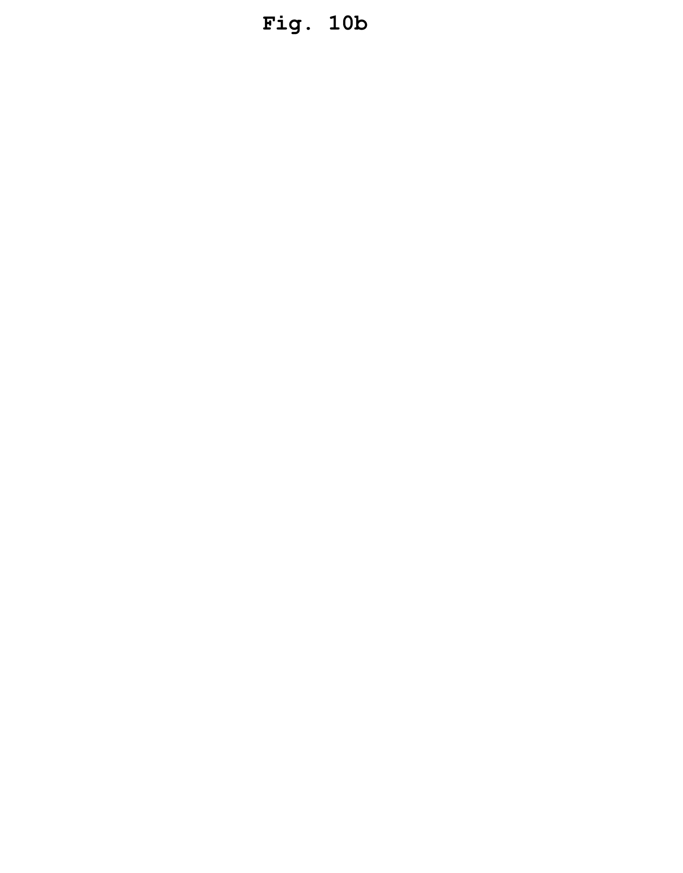

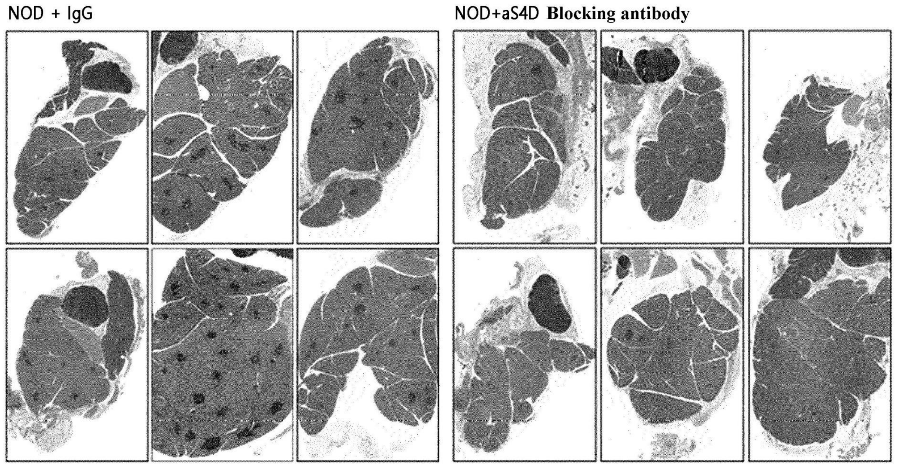

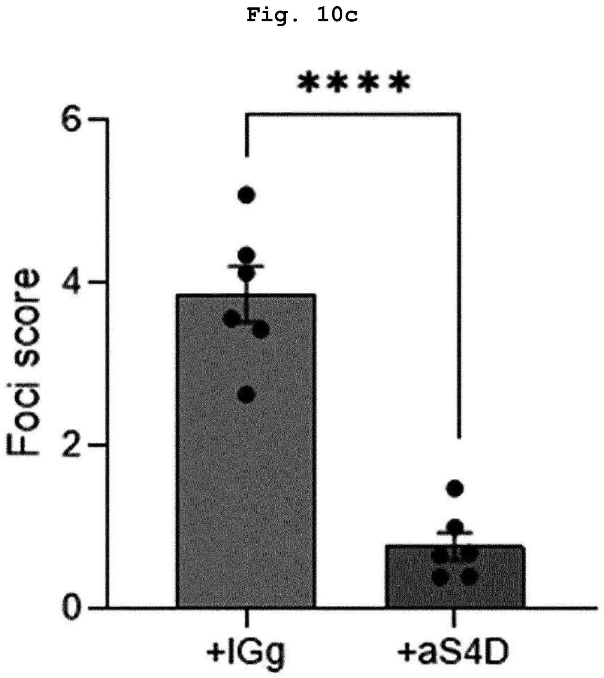

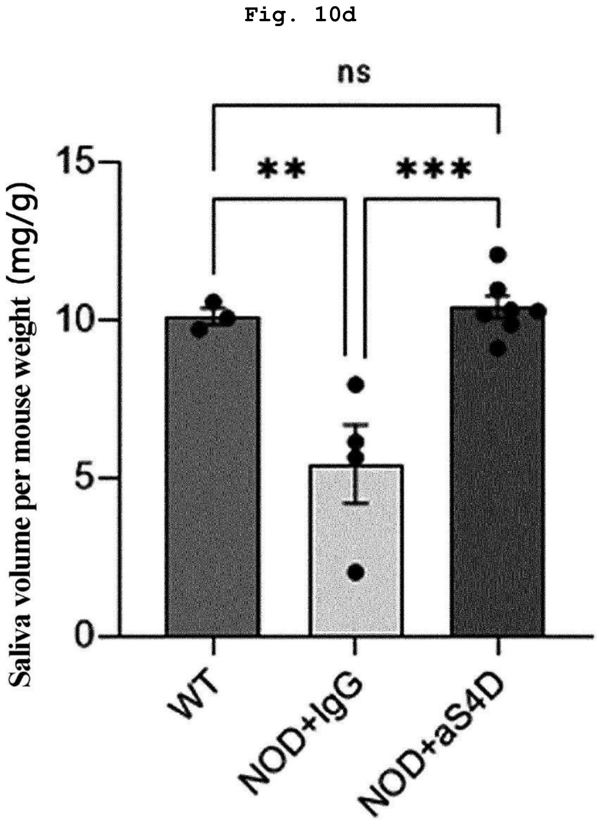

An anti-sema 4D blocking antibody (SC-390675, Santa Cruz) was locally injected directly into the submandibular gland of a 10-week NOD mouse model (injection with 20 µL of 120 ng/µL blocking antibody per submandibular gland), and collected on week 13, and then the examination was performed, and the results were shown in FIGS. 10A to 10F.

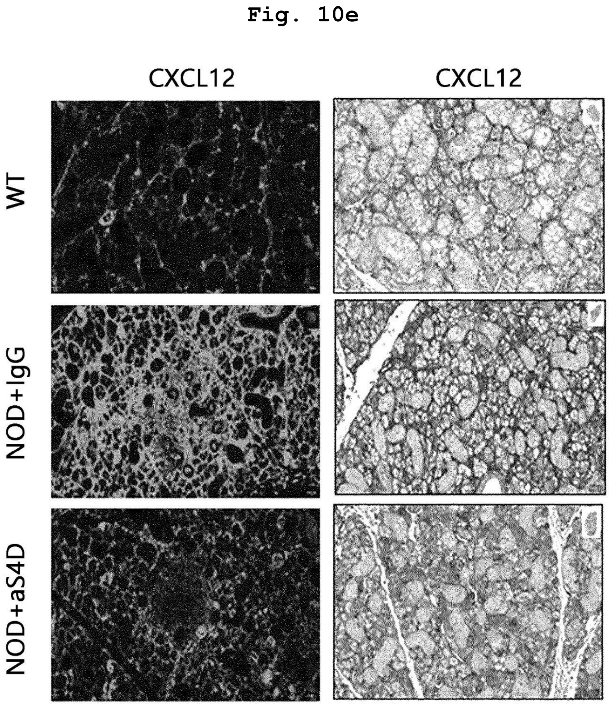

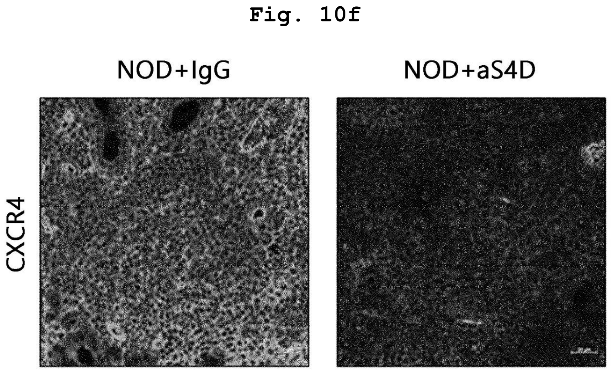

Referring to FIGS. 10A to 10D, it was confirmed that the infiltration of immune cells into the tissue was effectively inhibited, and the reduced amount of saliva was restored to a normal level. Referring to FIG. 10E, it could be confirmed that the increased expression level of CXCL12 compared to a WT mouse in the tissue was decreased in an anti-sema 4D blocking antibody treated group. Referring to FIG. 10F, it could be confirmed that the expression level of CXCR4 in the NOD mouse immune cell foci was also decreased in the anti-sema 4D blocking antibody treated group.

While exemplary embodiments of the present disclosure have been described in detail above, the scope of the present disclosure is not limited thereto, and various modifications and improvements made by those skilled in the art using the basic concept of the present disclosure defined in the following claims also fall within the scope of the present disclosure.

All technical terms used in the present disclosure, unless otherwise defined, are used as the meaning as commonly understood by those skilled in the related art of the present disclosure. The contents of all publications disclosed herein by reference are incorporated in the present disclosure.

## Claims

1. A pharmaceutical composition for preventing or treating Sjogren's syndrome, the pharmaceutical composition containing a recombinant anti-Semaphorin 4D antibody as an active ingredient.

2. The pharmaceutical composition for preventing or treating Sjogren's syndrome of claim 1, wherein the recombinant anti-Semaphorin 4D antibody inhibits the expression level of at least one gene selected from CXCL12, CXCR4 or a combination thereof.

3. The pharmaceutical composition for preventing or treating Sjogren's syndrome of claim 1, wherein the recombinant anti-Semaphorin 4D antibody blocks at least one protein selected from CD72, CD100, Plexin B2 or a combination thereof.

4. The pharmaceutical composition for preventing or treating Sjogren's syndrome of claim 1, wherein the composition is applied in the form of an injection.

5. A kit for diagnosing Sjogren's syndrome, the kit containing an anti-Semaphorin 4D antibody as an active ingredient.

6. A method for preventing or treating Sjogren's syndrome, the method comprising administering an effective amount of recombinant anti-Semaphorin 4D antibody to a subject in need thereof.

7. A use of a recombinant anti-Semaphorin 4D antibody for use in the preparation of a pharmaceutical agent for the prevention or treatment of Sjogren's syndrome.

8. A method for diagnosing Sjogren's syndrome, the method comprising measuring the expression or activity of semaphorin 4D by contacting an isolated subject sample with a recombinant anti-Semaphorin 4D antibody.

9. A use of a recombinant anti-Semaphorin 4D antibody for use in the preparation of an agent for the diagnosis of Sjogren's syndrome.
